# EUROPEAN PATENT SPECIFICATION

(11) **EP 4 431 148 B1**
(45) Date of publication and mention of the grant of the patent: **15.04.2026**
(21) Application number: 21964193.3
(22) Date of filing: 19.11.2021
(51) Int. Cl.: A61N 1/39, A61H 31/00, A61B 5/349

(54) **SYSTEM COMBINING CARDIOPULMONARY RESUSCITATION DEVICE AND AUTOMATED EXTERNAL DEFIBRILLATOR, AND ALGORITHM FOR CONTROLLING SAME**
SYSTEM MIT KOMBINATION AUS EINER KARDIOPULMONALEN WIEDERBELEBUNGSVORRICHTUNG UND EINEM AUTOMATISIERTEN EXTERNEN DEFIBRILLATOR SOWIE ALGORITHMUS ZUR STEUERUNG DAVON
SYSTÈME COMBINANT UN DISPOSITIF DE RÉANIMATION CARDIO-PULMONAIRE ET UN DÉFIBRILLATEUR EXTERNE AUTOMATISÉ, ET SON ALGORITHME DE COMMANDE

(30) Priority: 12.11.2021 KR 20210155872
(43) Date of publication of application: 18.09.2024
(73) Proprietor: CU Medical Systems Inc., Wonju-si, Gangwon-do 26365 (KR)
(72) Inventor: SONG, In-ho, Seoul 02799 (KR); KIM, Hyun-geun, Seoul 07524 (KR)
(74) Representative: Schiweck Weinzierl Koch Patentanwälte Partnerschaft mbB
(86) International application number: PCT/KR2021/017040
(87) International publication number: WO 2023/085483

(56) References cited:
- CN-A- 112 168 662
- CN-A- 113 197 766
- KR-A- 20130 015 898
- KR-A- 20170 122 680
- KR-B1- 101 950 028
- US-A1- 2019 255 340
- US-A1- 2021 283 009
- US-A1- 2021 283 009
- US-B2- 7 226 427

## Description

### [Technical Field]

The present invention relates to a system combining a cardiopulmonary resuscitation device and an automated external defibrillator, which is a combination of a cardiopulmonary resuscitation device and an automated external defibrillator, and an algorithm for controlling the same.

### [Background Art]

Cardiopulmonary resuscitation (CPR) is a procedure in which a series of processes such as chest compression, maintenance of an airway, and artificial respiration are repeated, and more specifically, when a patient is suspected of cardiac arrest, a person, who performs the CPR, confirms safety on scene, confirms a reaction and respiratory status of a collapsed person, asks for help from people around him/her, quickly reports to an emergency rescue organization, and then clasps both hands together, compresses a center (avoid a solar plexus, and center area of both nipples) of a chest with heels of hands and elbows 30 times, in which a compression depth is less than 4 to 6 cm, secures the airway after the chest compression, performs artificial respiration a total of 2 times, 1 time/1 to 2.5 seconds while checking whether the chest is inflated, and alternately performs the chest compressions and the artificial respiration twice.

However, even if the general public has received CPR training, there is a problem in that it is difficult to accurately determine the compression depth due to the unfamiliarity of CPR and the burden of rib damage and the inconsistent chest height for each patient in the event of cardiac arrest.

Accordingly, various types of cardiopulmonary resuscitation (CPR) devices are known. One of the devices is driven by compressed air or breathing gas (Jolife AB; Lucas TM, Lund, Sweden). A unique advantage of the cardiopulmonary resuscitation device is that it is light in weight and can be transported. Another advantage of the cardiopulmonary resuscitation device is the elastic nature of compressed air, and thus, gas-powered CPR devices cause less damage to the chest of the patient than devices equipped with rigid compression means. The known device may be used as an emergency facility in life-saving situations. In addition, the known device may be supplied with drive gas from a hospital air supply line, which may be desirable for uninterrupted blows of CPR when a patient is hospitalized.

However, the cardiopulmonary resuscitation device has a problem in that it is difficult to accurately determine whether the cardiac arrest of the patient occurs because it does not have a function for analyzing a heart rhythm (electrocardiogram (ECG)).

Meanwhile, an automated external defibrillator (AED) is known as a device for analyzing a heart rhythm. The automated external defibrillator is a device that removes cardiac fibrillation semi-automatically. When a sudden cardiac arrest (SCA) phenomenon in which the supply of oxygen, etc., essential for vital activities is stopped as the heartbeat stops and a blood flow to each tissue is stopped is confirmed through heart rhythm analysis, the automated external defibrillator gives an electric shock from the outside to return the heartbeat of the patient to normal.

However, since the automated external defibrillator can only provide cardiac shock based on the electric shock, the automated external defibrillator has poor versatility as it cannot be used on patients at risk. Therefore, the automated external defibrillator is difficult to use on patients at risk, making it difficult to get the heartbeat to return to normal.

The related prior art is as follows.
CN 113 197 766A describes an emergency clinical adjustable cardiopulmonary resuscitation device comprising a guide rail frame, wherein the guide rail frame is a frame body formed by fixedly connecting two parallel guide rails end to end, a center seat is fixed in the middle of the guide rail frame, a vertical center piston assembly is fixed in the center seat, and a pressing gasket is fixed below the center piston assembly; two symmetrically-arranged sliding arms are slidably arranged below the guide rail frame, side pressure shoe assemblies are symmetrically arranged in the opposite directions of the two sliding arms, and a side piston is arranged between each side pressure shoe assembly and each sliding arm.
US 2021/283009A1 describes an adjustable chest compression device that can adjust to accommodate a variety of patient sizes, whrein the chest compression device can include adjustable support legs structured to support the chest compression mechanism at a distance from the base member and adjust to accommodate a patient size.
US 7 226 427B2 describes a method for cardiopulmonary resuscitation of a person, comprising providing a breathing gas under positive pressure to the airways of the person; mechanically stimulating the person's heart using a pneumatic drive unit to reciprocate a rod for compressing and decompressing the person's chest while inducing the person to inspire and expire against the positive pressure of the breathing gas for at least a period of time until a positive coronary perfusion pressure develops; and
electrically stimulating the heart once the positive coronary perfusion pressure exceeds a threshold pressure, wherein the pneumatic drive unit is driven by a gas that is recycled for use as the breathing gas; and a system for administering steps in a course of cardiopulmonary resuscitation to a person, comprising a chest compression device for administering mechanical stimulation to the person's heart using a pneumatic drive unit to reciprocate a rod for compressing and decompressing the person's chest; a module for administering electric stimulation to the heart; a control unit for coordinating the administration of mechanical and electric stimulation to the heart over the course of the cardiopulmonary resuscitation; and means for supplying pressurized breathing gas to the person's lungs during the course of the cardiopulmonary resuscitation, wherein the pneumatic drive unit operates on a gas, and wherein the means for supplying the pressurized breathing gas recycles the gas that is used to operate the pneumatic drive unit. CN 112 168 662A describes a clinical multi-functional rescue device of using of emergency surgery, including the underframe frame, a pair of first slide rail of inside wall fixedly connected with of underframe frame, the surface of first slide rail is provided with a plurality of first sliders, through first bracing piece fixed connection between the first slider, the surface of first bracing piece is provided with a pair of lifter, the top fixedly connected with bed board of lifter, the lateral wall symmetry of bed board is provided with a pair of first mounting panel, a plurality of second bracing pieces of fixed surface of first mounting panel are connected with, the top fixedly connected with top frame of second bracing piece, the inside wall of top frame is provided with a pair of first lead screw, the surface of first lead screw all is provided with first motion piece. The spatial position of the installation box is adjusted by controlling the first driving motor, the second driving motor and the second electric push rod to work.

### [Disclosure]

### [Technical Problem]

Therefore, the present invention has been made to solve the above problems, and an object of the present invention provides an algorithm for controlling a system combining a cardiopulmonary resuscitation device and an automated external defibrillator that analyzes a heart rhythm of a patient during emergency treatment of the patient, and performs a chest compression and an electric shock simultaneously or performs only chest compression according to the heart rhythm analysis result to return a patient's heartbeat to normal.

In addition, an object of the present invention provides an algorithm for controlling a system combining a cardiopulmonary resuscitation device and an automated external defibrillator in which a chest compression unit for compressing a chest of a patient can slidably move to accurately compress the patient's chest.

However, objects of the present invention are not limited to the above-described objects. That is, other objects that are not described may be obviously understood by those skilled in the art to which the present invention pertains from the following description.

### [Technical Solution]

To accomplish the above object, according to an exemplary embodiment of the present invention, an algorithm for controlling a system combining a cardiopulmonary resuscitation device and an automated external defibrillator includes: a first step in which a control unit determines whether the system combining a cardiopulmonary resuscitation device and an automated external defibrillator is set to an automatic heart shock mode and/or a chest compression mode; a second step in which the control unit determines whether a plurality of electrodes or pads of an electrocardiogram measurement unit is attached to the chest of a patient; a third step in which a rhythm determination and shock signal generation unit analyzes a heart rhythm of the patient through the electrocardiogram measurement unit to determine whether the heart rhythm is a shockable rhythm or a nonshockable rhythm; a fourth step in which, after the heart rhythm of the patient is analyzed, a chest compression unit is lowered to be in contact with the chest of the patient and presses the chest of the patient; a fifth step in which the control unit determines whether an input signal is input to a shock button when the rhythm determination and shock signal generation unit determines that the heart rhythm of the patient is a shockable rhythm; a sixth step in which, when an input signal is input to the shock button, an electric shock unit generates high voltage energy through the rhythm determination and shock signal generation unit to apply an electric shock to the patient; and a seventh step in which, when the control unit determines that no input signal is input to the shock button, the control unit controls the electric shock unit to internally discharge the high voltage energy.

The first step may include: a 1-1-th step in which a control button of the control unit is input; a 1-2-th step in which the control unit determines whether an input signal is input to a rhythm analysis button for operating the electrocardiogram measurement unit and the rhythm determination and shock signal generation unit; and a 1-3-th step in which the control unit determines whether the input signal is input to the shock button.

The first step may further include a 1-4-th step in which the control unit controls the chest compression treatment method for the patient when it is determined that the input signal is not input to the shock button.

The 1-4-th step may include: a step of setting the compression mode of the chest compression unit to a compression continuous mode continuously compressing the chest of the patient or a compression 30 : 2 mode simultaneously performing the chest compression 30 times and artificial respiration twice on the patient through a compression mode setting button of the control unit; a step of setting a compression depth of the chest compression unit through a compression depth setting button of the control unit; and a step of setting a compression speed of the chest compression unit through a compression speed setting button of the control unit.

In the fourth step, the chest compression unit may compress the chest of the patient at the compression depth and compression speed set through the compression depth setting button and the compression speed setting button based on the compression continuous mode or the compression 30 : 2 mode set through the compression mode setting button.

The third step may include: a step of detecting, by the electrocardiogram measurement unit, an electrocardiogram signal from the patient through the plurality of electrodes or pads attached to the chest of the patient, amplifying the detected electrocardiogram signal, removing noise from the amplified electrocardiogram signal, and then converting the electrocardiogram signal into a digital signal; and a step of analyzing, by the rhythm determination and shock signal generation unit, the heart rhythm of the patient based on the digital signal received from the electrocardiogram measurement unit.

The sixth step may include: a step of receiving, by the electric shock unit, from the rhythm determination and shock signal generation unit, an electric shock signal generated when the rhythm determination and shock signal generation unit determines that the heart rhythm of the patient is a shockable rhythm; and a step of generating, by the electric shock unit, high voltage energy based on the electric shock signal to apply an electric shock to the patient when the input signal is input to the shock button.

In the seventh step, when a voice guide is output from an alarm unit to inform that the input signal is not input to the shock button, the electric shock unit may internally discharge the high voltage energy.

In the seventh step, when the input signal is not input to the shock button, from the alarm unit, the voice guide is output to induce the input of the input signal to the shock button, and then the voice guide indicating that the automated external defibrillator mode is canceled is output, the electric shock unit may internally discharge the high voltage energy.

In an algorithm for controlling a system combining a cardiopulmonary resuscitation device and an automated external defibrillator, the chest compression and electric shock may be alternately or simultaneously applied to the patient depending on operation timing of the electric shock unit and the chest compression unit.

In the repetitive process of measuring the electrocardiogram of the patient, determining the rhythm, providing the chest compression and the electric shock, and internally discharging the high voltage energy, the control unit may control the chest compression unit to stop the chest compression immediately before the rhythm determination and shock signal generation unit determines the rhythm and to continuously perform the chest compression immediately after the rhythm determination and shock signal generation unit determines the rhythm.

According to an exemplary embodiment of the present invention, a system combining a cardiopulmonary resuscitation device and an automated external defibrillator includes: a support plate that supports a back of a patient; a support whose one end and the other end are coupled to both edges of the support plate; and a hood that is coupled to one side of the support, connected to a chest compression unit for compressing a chest of the patient, and provided with a control unit equipped with a shock button, in which the support plate includes: an electrocardiogram measurement unit that detects an electrocardiogram signal from the patient, amplifies the detected electrocardiogram signal, removes noise from the amplified electrocardiogram signal, and then converts the electrocardiogram signal into a digital signal; when receiving the digital signal from the electrocardiogram measurement unit, a rhythm determination and shock signal generation unit that analyzes the heart rhythm of the patient with the digital signal to determine whether the heart rhythm of the patient is a shockable rhythm or a nonshockable rhythm, and generates an electric shock signal when the heart rhythm of the patient is the shockable rhythm; and when receiving the electric shock signal from the rhythm determination and shock signal generation unit, an electric shock unit that emits voltage energy to electrodes or pads attached to the chest of the patient to apply an electric shock to the patient.

The control unit may control at least one of the chest compression unit, the electrocardiogram measurement unit, the rhythm determination and shock signal generation unit, and the electric shock unit to operate.

In the repetitive process of measuring the electrocardiogram of the patient, determining the rhythm, providing the chest compression and the electric shock, and internally discharging the high voltage energy, the control unit may control the chest compression unit to stop the chest compression immediately before the rhythm determination and shock signal generation unit determines the rhythm and to continuously perform the chest compression immediately after the rhythm determination and shock signal generation unit determines the rhythm.

In a system combining a cardiopulmonary resuscitation device and an automated external defibrillator according to the exemplary embodiment of the present invention, the chest compression and electric shock may be alternately or simultaneously applied to the patient depending on operation timing of the electric shock unit and the chest compression unit.

### [Advantageous Effects]

The present invention has excellent versatility as it is possible to provide a chest compression and an electric shock to a patient with one system through a combination of a cardiopulmonary resuscitation device and an automated external defibrillator.

In addition, the present invention can accurately compress a chest of a patient through the sliding movement of the cardiopulmonary resuscitation device.

However, effects which can be achieved by the present invention are not limited to the above-described effects. That is, other objects that are not described may be obviously understood by those skilled in the art to which the present invention pertains from the following description.

### [Description of Drawings]

FIG. 1 is a perspective view of a system combining a cardiopulmonary resuscitation device and an automated external defibrillator according to an exemplary embodiment of the present invention.
FIG. 2 is a schematic diagram of the system combining a cardiopulmonary resuscitation device and an automated external defibrillator according to the exemplary embodiment of the present invention.
FIG. 3 is a block diagram illustrating a control unit according to an exemplary embodiment of the present invention.
FIG. 4 is a diagram illustrating an algorithm for controlling a system combining a cardiopulmonary resuscitation device and an automated external defibrillator according to an exemplary embodiment of the present invention.
FIG. 5 is a diagram illustrating chest compression treatment settings in the control algorithm illustrated in FIG. 4.

### [Best Mode]

Hereinafter, exemplary embodiments of the present invention will be described in detail with reference to the accompanying drawings so that those skilled in the art to which the present invention pertains may easily practice the present invention. However, description of the present invention is only an exemplary embodiment for structural or functional description, and therefore the scope of the present invention should not be construed as limited to exemplary embodiments described in the text. That is, since the exemplary embodiments may be variously modified and may have various forms, the scope of the present invention should be construed as including equivalents capable of realizing the technical idea. In addition, a specific exemplary embodiment is not construed as including all the objects or effects presented in the present invention or only the effects, and therefore the scope of the present invention should not be understood as being limited thereto.

The meaning of the terms described in the present specification should be understood as follows.

Terms such as "first" and "second" are intended to distinguish one component from another component, and the scope of the present invention should not be limited by these terms. For example, a first component may be named a second component and the second component may also be similarly named the first component. It is to be understood that when one element is referred to as being "connected to" another element, it may be connected directly to or coupled directly to another element or be connected to another element, having the other element intervening therebetween. On the other hand, it is to be understood that when one element is referred to as being "connected directly to" another element, it may be connected to or coupled to another element without the other element intervening therebetween. Meanwhile, other expressions describing a relationship between components, that is, "between", "directly between", "neighboring to", "directly neighboring to" and the like, should be similarly interpreted.

It should be understood that the singular expression includes the plural expression unless the context clearly indicates otherwise, and it will be further understood that the terms "comprises" or "have" used in this specification, specify the presence of stated features, numerals, steps, operations, components, parts, or a combination thereof, but do not preclude the presence or addition of one or more other features, numerals, steps, operations, components, parts, or a combination thereof.

Unless defined otherwise, all the terms used herein including technical and scientific terms have the same meaning as meanings generally understood by those skilled in the art to which the present invention pertains. It should be understood that the terms defined by the dictionary are identical to the meanings within the context of the related art, and they should not be ideally or excessively formally defined unless the context clearly dictates otherwise.

### Configuration of Exemplary Embodiment

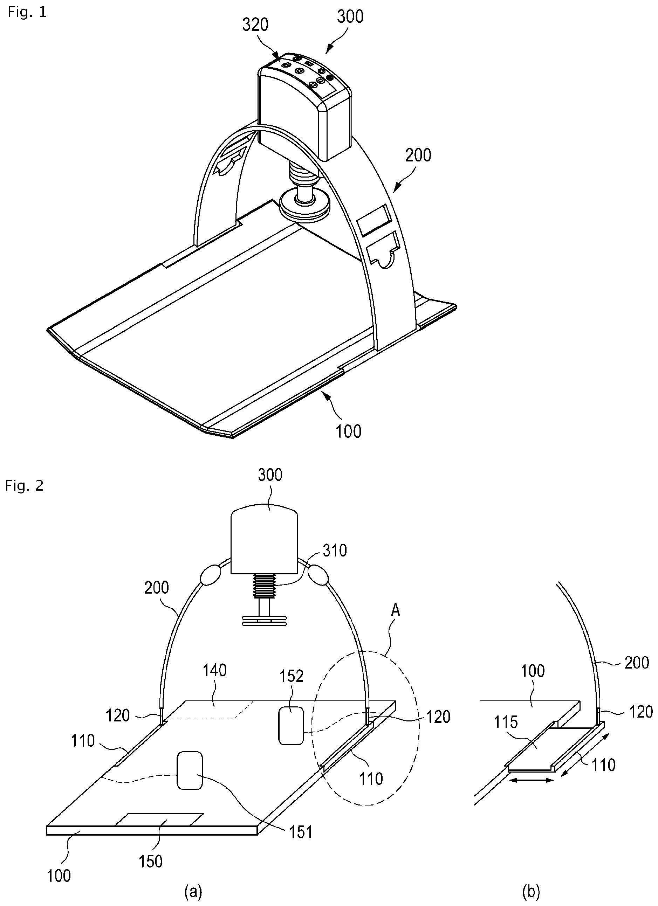

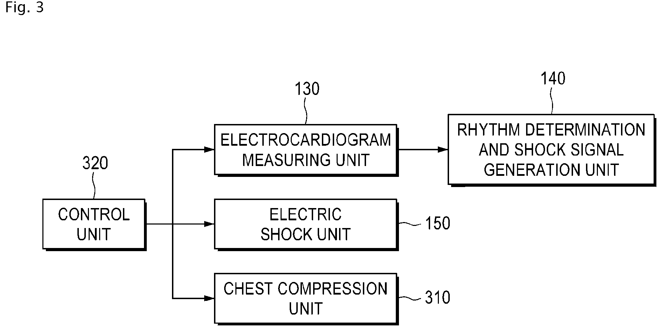

FIG. 1 is a perspective view of a system combining a cardiopulmonary resuscitation device and an automated external defibrillator according to an exemplary embodiment of the present invention, FIG. 2 is a schematic diagram of the system combining a cardiopulmonary resuscitation device and an automated external defibrillator according to the exemplary embodiment of the present invention, and FIG. 3 is a block diagram illustrating a control unit according to an exemplary embodiment of the present invention.

Referring to FIGS. 1 to 3, the system combining a cardiopulmonary resuscitation device and an automated external defibrillator of the present invention includes a support plate 100, a support 200, and a hood 300.

The support plate 100 is formed in a form for supporting a back of a patient (sudden cardiac arrest, ventricular fibrillation, etc.).

The support plate 100 includes a sliding guide 110 for sliding and moving the support 200 and the hood 300 which are the cardiopulmonary resuscitation device, and a stopper 120 for fixing positions of the support 200 and the hood 300.

In addition, a side portion of the support plate 100 is provided with an internal space, into which a frame 115 provided on the sliding guide 110 can be drawn, to adjust a height of a chest compression unit 310.

The sliding guide 110 is provided on both edges of the support plate 100, and one end and the other end of the support 200 are coupled to be slidably movable so that the support 200 slides forward or backward.

As illustrated in (b) of FIG. 2 which is enlarged area A illustrated in (a) of FIG. 2, the sliding guide 110 is provided with the frame 115, which is drawn into the inside of the support plate 100 or drawn out of the inside of the support plate 100, to adjust a distance between both ends of the support 200 and adjust a height of the chest compression unit 310.

In this case, the reason for adjusting the height of the chest compression unit 310 is to prevent a situation in which the chest compression unit 310 may not compress a chest of a specific patient from occurring because each patient has different body shapes.

The stopper 120 is provided on the sliding guide 110 and is coupled to one end and the other end of the support 200 as it is formed in a form capable of being coupled to one end and the other end of the support 200, and fixes the positions of the support 200 and the hood 300 through the coupling to one end and the other end of the support 200.

An electrocardiogram measuring unit 130, a rhythm determination and shock signal generation unit 140, and an electric shock unit 150 are further provided on the support plate 100 to provide treatment through electric shock to the patient.

The electrocardiogram measurement unit 130 is connected to the rhythm determination and shock signal generation unit 140 and a control unit 320 to be described later, and when operating by the control unit 320, detects and then amplifies the electrocardiogram signal from the patient, removes noise from the amplified ECG signal, and converts the ECG signal into a digital signal, and transmits the digital signal to the rhythm determination and shock signal generation unit 140.

When an analog electrocardiogram signal is detected, the electrocardiogram measurement unit 130 may include an amplifier that amplifies the detected analog electrocardiogram signal, a filter that removes noise from the electrocardiogram signal amplified by the amplifier, and an A/D converter that converts the electrocardiogram signal from which the noise has been removed by the filter into the digital signal.

The electrocardiogram measurement unit 130 is composed of electrodes built into the support plate 100 or pads attachable to the patient in order to detect the analog electrocardiogram signal from the patient, and detects the electrocardiogram signal from a patient whose back is supported on a support plate 100.

Here, since a process of detecting the electrocardiogram signal of the patient through the plurality of electrodes or pads is general, a description thereof will be omitted for convenience.

The rhythm determination and shock signal generation unit 140 is built into the support plate 100, and is connected to the electrocardiogram measurement unit 130 to operate together when the electrocardiogram measurement unit 130 operates by the control unit 320, and thus receives the digital signal from the electrocardiogram measurement unit 130.

When receiving the digital signal from the electrocardiogram measurement unit 130, the rhythm determination and shock signal generation unit 140 analyzes a heart rhythm of a patient using a digital signal to determine whether the heart rhythm of the patient is shockable rhythm or nonshockable rhythm, when it is determined that the heart rhythm of the patient is the shockable rhythm, generates an electric shock signal, and when the electric shock unit 150 operates, transmits an electric shock signal to the electric shock unit 150.

The electric shock unit 150 is connected to the rhythm determination and shock signal generation unit 140, and when it is determined that the heart rhythm of the patient is the shockable rhythm from the rhythm determination and shock signal generation unit 140, receives an electric shock signal from the rhythm determination and shock signal generation unit 140 when operating by the control unit 320.

Hereinafter, the description will be made based on the fact that the electric shock unit 150 has a plurality of electrodes.

When the electric shock unit 150 receives the electric shock signal from the rhythm determination and shock signal generation unit 140, the electric shock unit 150 generates high voltage energy so that the high voltage electric shock is generated from each of a first defibrillation electrode 151 and a second defibrillation electrode 152 and emits the generated high voltage energy to the first defibrillation electrode 151 and the second defibrillation electrode 152.

The first defibrillation electrode 151 and the second defibrillation electrode 152 are preferably attached to the chest of the patient so that the electric shock is applied to the patient. As a specific example, the first defibrillation electrode 151 may be attached below a right clavicle of a patient to apply the electric shock to the patient, and the second defibrillation electrode 152 may be attached to an armpit next to a left nipple of a patient to provide the electric shock to the patient.

The first defibrillation electrode 151 and the second defibrillation electrode 152 may be equipped with a sensor (not illustrated) that detects whether or not they are attached to the chest of the patient, and transmit data on whether the sensor (not illustrated) is in contact with the chest of the patient to the control unit 320.

The support 200 and the hood 300 are provided in the system combining a cardiopulmonary resuscitation device and an automated external defibrillator of the present invention to operate as the cardiopulmonary resuscitation device that compresses the chest of the patient.

The support 200 is combined with a sliding guide 110 to allow the chest compression unit 310, which will be described later, to move to a position that compresses the chest of the patient, and in the present invention, the shape for supporting the hood 300 may have an arch shape, but it is not limited thereto.

As one end and the other end of the support 200 are movably coupled to a pair of sliding guides 110, the support 200 may move forward or backward around the sliding guide 110 as an axis, or drawn into and out of the frame 115 to adjust a distance between both ends.

In this case, it is preferable that the support 200 moves forward and backward and adjusts the distance between both ends before the chest compression unit 310 compresses the chest of the patient. When the chest compression unit 310 has moved to a position where it compresses the chest of the patient by the user providing first aid to the patient, one end and the other end are coupled by the pair of stoppers 120, so the chest compression unit 310 is fixed in the position where it compresses the chest of the patient.

In addition, the support 200 has one end and the other end detachable from the pair of sliding guides 110 so that it can be detached from the pair of sliding guides 110, and through the detachment, the support 200 and the hood 300 are detachable from the support plate 100 to enable the separate use of the cardiopulmonary resuscitation device.

The hood 300 is coupled to one side of the support 200, more specifically to an arch crown of the arch-shaped support 200, and is connected to the chest compression unit 310 for compressing the chest of the patient, and includes the control unit 320 for controlling the operation of the system combining a cardiopulmonary resuscitation device and an automated external defibrillator.

The chest compression unit 310 is spaced apart from the chest of the patient before compressing the chest of the patient, and is operated by the control unit 320 to be lowered to the position where it compresses the chest of the patient and then compress the chest of the patient.

The chest compression unit 310 compresses the chest of the patient in a compression continuous mode that continuously compresses the chest of the patient or a compression 30 : 2 mode that performs the compression of the chest of the patient 30 times and then artificial respiration twice depending on the chest compression mode to provide the emergency treatment based on the chest compression to the patient.

The chest compression unit 310 may be operated alternately or simultaneously with the electric shock unit 150.

In other words, the system combining a cardiopulmonary resuscitation device and an automated external defibrillator of the present invention has excellent versatility as it is possible to alternately or simultaneously provide the chest compression and the electric shock to a patient with one system according to the operation timing of the electric shock unit 150 and the electric shock unit 310.

The control unit 320 may be provided with a plurality of buttons to operate at least one of the chest compression unit 310, the electrocardiogram measurement unit 130, the rhythm determination and shock signal generation unit 140, and the electric shock unit 150.

As a specific example, the plurality of buttons may include a power button for turning on/off the system combining a cardiopulmonary resuscitation device and an automated external defibrillator of the present invention, a control button for setting the mode of the system combining a cardiopulmonary resuscitation device and an automated external defibrillator to the automated external defibrillator mode or the chest compression mode, a stop button for stopping the operation of the chest compression unit 310, a compression mode setting button for allowing the chest compression unit 310 to perform a chest compression (CPR) or setting the chest compression mode of the chest compression unit 310, a compression depth setting button for setting the chest compression depth of the chest compression unit 310, a compression speed setting button for setting the chest compression speed (number of times) of the chest compression unit 310, a rhythm analysis button for operating the electrocardiogram measurement unit 130 and the rhythm determination and shock signal generation unit 140, and a shock button for operating the electric shock unit 150.

When the input signal is input to the power button and the system combining a cardiopulmonary resuscitation device and an automated external defibrillator of the present invention is turned on, the control unit 320 initializes settings and performs a self-test to determine whether the normal operation is possible, and if the input signal is input to the power button when the system combining a cardiopulmonary resuscitation device and an automated external defibrillator of the present invention is turned on, the control unit 320 initializes settings and makes the power supply of the system combining a cardiopulmonary resuscitation device and an automated external defibrillator of the present invention into a turn-off state.

The control unit 320 may control the chest compression unit 310 so that the chest of the patient is compressed when the compression mode setting button is in the compression continuous mode, and in contrast, when the compression mode setting button is not in the compression continuous mode, the control unit may control the chest compression unit 310 so that the chest of the patient is compressed 30 times and then the artificial respiration is performed twice.

When the input signal is input to the compression depth setting button, the control unit 320 may control the chest compression unit 310 so that the chest of the patient is compressed to a depth of at least one of 4 cm, 4.5 cm, 5 cm, and 5.5 cm.

Furthermore, the control unit 320 may control the chest compression unit 310 so that when the input signal is input to the compression depth setting button in the initialization state, the chest of the patient is compressed by 5 cm, when the signal is input, the chest of the patient is compressed by 5.5 cm, when the signal is input again, the chest of the patient is compressed by 4 cm, and when the signal is input again, the chest of the patient is compressed by 4.5 cm.

The control unit 320 may control the chest compression unit 310 so that when the input signal is input to the compression speed setting button, the chest of the patient is compressed at least one of 100, 110, and 120 times.

Furthermore, the control unit 320 may control the chest compression unit 310 so that, when the input signal is input to the compression speed setting button in the initialization state, the chest of the patient is compressed 110 times, when the signal is input again, the chest of the patient is compressed 120 times, and when the signal is input again, the chest of the patient is compressed 100 times.

### Operation of Exemplary Embodiment

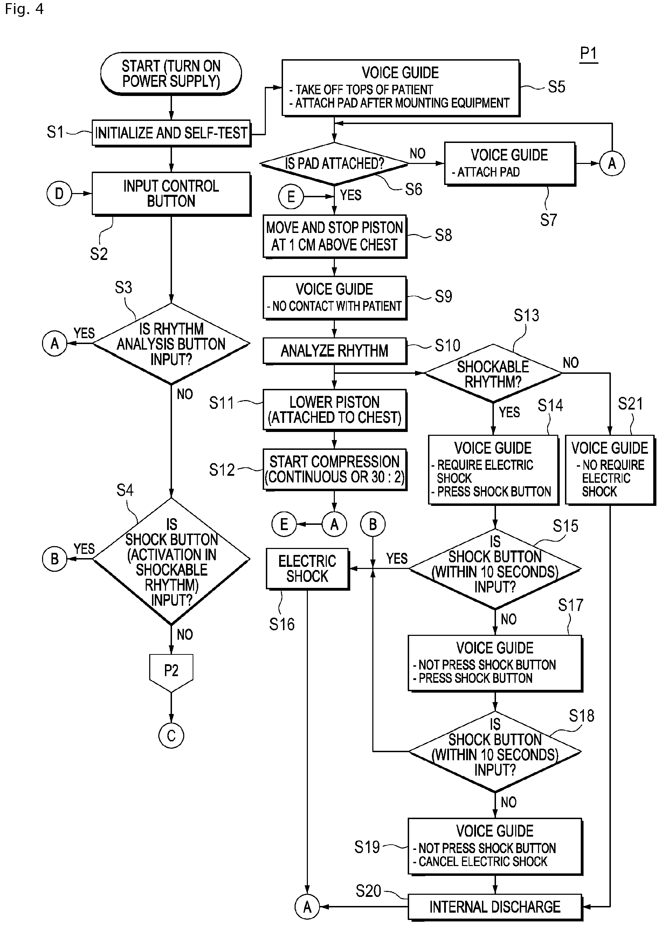

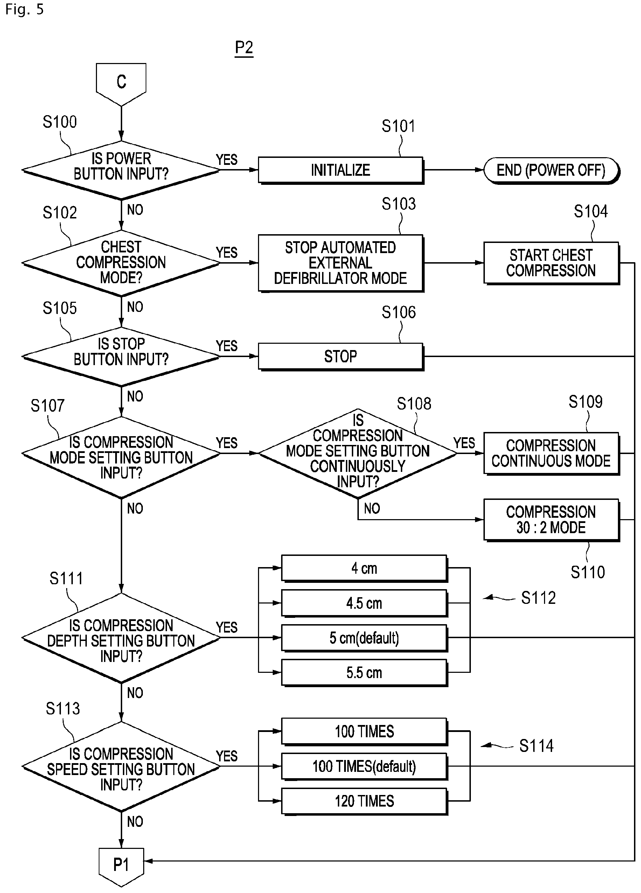

FIG. 4 is a diagram illustrating an algorithm for controlling a system combining a cardiopulmonary resuscitation device and an automated external defibrillator according to an exemplary embodiment of the present invention, and FIG. 5 is a diagram illustrating chest compression treatment settings in the control algorithm illustrated in FIG. 4.

The control algorithm illustrated in FIGS. 4 and 5 may be performed to control the system combining a cardiopulmonary resuscitation device and an automated external defibrillator of the present invention. Therefore, even if the contents are omitted below, it is preferable to understand that the operational flow process is achieved by the system combining a cardiopulmonary resuscitation device and an automated external defibrillator of the present invention.

Referring to FIG. 4, in order for the system combining a cardiopulmonary resuscitation device and an automated external defibrillator of the present invention to provide the chest compression treatment and the electric shock treatment to the patients, when the input signal is input to the power button and the power supply is turned on, the control unit 320 may initialize the settings of the system combining a cardiopulmonary resuscitation device and an automated external defibrillator of the present invention and perform a self-test to determine whether the system combining a cardiopulmonary resuscitation device and an automated external defibrillator of the present invention can operate normally (S1).

After the initialization and self-test, the control button of the control unit 320 for controlling the mode of the system combining a cardiopulmonary resuscitation device and an automated external defibrillator of the present invention to the automated external defibrillator mode may be input by the user (S2).

After inputting the control button, the control unit 320 may determine whether the input signal is input to the rhythm analysis button (S3).

In this case, when it is determined that the rhythm analysis button is input (S3-YES), the control unit 320 operates the electrocardiogram measurement unit 130 and the rhythm determination and shock signal generation unit 140, and in contract, when it is determined that the rhythm analysis button is not input (S3-NO), the control unit 320 may determine whether the shock button is input by the user (S4).

In this case, when it is determined that the shock button is input (S4-YES), the control unit 320 operates the electric shock unit 150 to provide the electric shock to the patient, and in contrast, when it is determined that the shock button is not input (S4-NO), the control unit 320 may control the chest compression treatment method of the system combining a cardiopulmonary resuscitation device and an automated external defibrillator of the present invention through the operation flow illustrated in FIG. 5.

Meanwhile, when the initialization and self-test are completed, the alarm unit built into the system combining a cardiopulmonary resuscitation device and an automated external defibrillator of the present invention may output a voice guide instructing to take off tops of the patient and attach a pad to the patient (S5).

In addition, the voice guide is not limited to the voice guide instructing to attach the pad, and when the electrocardiogram measurement unit 130 is composed of a plurality of electrodes, the voice guide instructing to attach the plurality of electrodes may be output, but in the exemplary embodiment, the description will be made based on the pad.

After outputting the voice guide, the user may attach the pad to the chest of the patient (S6).

In this case, when the control unit 320 determines that the pad is not attached to the chest of patient, the alarm unit may again output the voice guide instructing to attach the pad to the chest of the patient (S7).

On the other hand, when the control unit 320 determines that the pad is attached to the chest of the patient (S6-YES), the chest compression unit 310 may be lowered to the initial position for compressing the chest of the patient (S8). In this case, the initial position may be a position where a lower end of the chest compression unit 310 and the chest of the patient are spaced apart by 1 cm.

After the chest compression unit 310 is lowered, the alarm unit may output the voice guide to warn the user not to touch the patient during the treatment process of the patient (S9).

After outputting the voice guide, the rhythm determination and shock signal generation unit 140 may analyze the heart rhythm of the patient based on the digital signal received from the electrocardiogram measurement unit 130 (S10).

After analyzing the heart rhythm of the patient, the chest compression unit 310 is lowered to contact the chest of the patient (S11), and the chest compression unit 310 may provide the chest compression treatment by compressing the chest of the patient (S12).

Meanwhile, when the heart rhythm of the patient is analyzed, the rhythm determination and shock signal generation unit 140 may determine whether the heart rhythm of the patient is the shockable rhythm (S13).

In this case, when it is determined that the heart rhythm of the patient is the shockable rhythm (S13-YES), the alarm unit may output the voice guide instructing to input the shock button because the electric shock treatment is required for the patient (S14).

After outputting the voice guide, the control unit 320 may determine whether the input signal is input to the shock button within a time (e.g., 10 seconds) preset by the user after the voice guide is output (S15).

In this case, when it is determined that the input signal is not input to the shock button within the preset time (S15-NO), the alarm unit may output the voice guide instructing to input the input signal to the shock button because the input signal is not input to the shock button.

After outputting the voice guide, the control unit 320 may again determine whether the input signal is input to the shock button within a time (e.g., 10 seconds) preset by the user (S18).

In this case, when it is determined that the input signal is not input to the shock button within the preset time (S18-NO), the alarm unit may output the voice guide instructing to cancel the automatic cardiac shock because the input signal is not input to the shock button.

At the same time, the control unit 320 may control the electric shock unit 150 so that the high voltage energy generated by the electric shock unit 150 is internally discharged at the same time that the voice guide is output (S20).

Meanwhile, when it is determined that the heart rhythm of the patient is the nonshockable rhythm rather than the shockable rhythm (S13-NO), the alarm unit may output the voice guide indicating that the electric shock treatment process is omitted, and the control unit 320 may control the electric shock unit 150 so that the high voltage energy generated by the electric shock unit 150 is internally discharged by omitting the electric shock treatment process (S20).

Here, except while the rhythm determination and shock signal generation unit 140 determines the rhythm in the repetitive process of measuring the electrocardiogram of the patient, determining the rhythm, providing the chest compression and the electric shock and internally discharging the high voltage energy, the control unit 320 controls the chest compression unit 310 to stop the chest compression immediately before determining the rhythm and continuously perform the chest compression immediately after determining the rhythm.

Referring to FIG. 5, in order for the system of the present invention combining a cardiopulmonary resuscitation device and an automated external defibrillator to control the chest compression treatment method for the patient, the control unit 320 may determine whether the input signal is input to the power button while the system combining a cardiopulmonary resuscitation device and an automated external defibrillator of the present invention is operating (S100).

In this case, when it is determined that the input signal is input to the power button (S100-YES), the control unit 320 may initialize the automated external defibrillator mode and chest compression mode (S101), and end the operation of the system combining a cardiopulmonary resuscitation device and an automated external defibrillator of the present invention.

On the other hand, when it is determined that the input signal is not input to the power button (S100-NO), the control unit 320 may determine whether the system combining a cardiopulmonary resuscitation device and an automated external defibrillator of the present invention is set to the chest compression mode based on that the input signal is input to the control button (S102).

In this case, when the system combining a cardiopulmonary resuscitation device and an automated external defibrillator of the present invention is set to the chest compression mode through the control button (S102-YES), the control unit 320 may control the automated external defibrillator mode in which the electric shock is performed to stop (S103) and the chest compression unit 310 to compress the chest of the patient (S104).

On the other hand, when it is determined that the input signal is not input to the control button (S102-NO), the control unit 320 may determine whether the input signal is input to the stop button (S105), and when it is determined that the input signal is input to the stop button, the control unit 320 may stop the operation of the chest compression unit 310 (S106).

In addition, when the input signal is not input to the stop button (S105-NO), the control unit 320 may determine whether the input signal is input to the compression mode setting button (S107).

In this case, when it is determined that the input signal is input to the compression mode setting button (S107-YES), the control unit 320 may determine whether the compression mode setting button is the compression continuous mode (S108), and when it is determined that the compression mode setting button is the compression continuous mode (S108-YES), the control unit 320 may control the chest compression unit 310 to operate in the compression continuous mode to continuously compress the chest of the patient (S109), and in contrast, when the compression mode setting button is not in the compression continuous mode, the control unit 320 may control the chest compression unit 310 to operate in the compression 30 : 2 mode in which the chest of patient is compressed 30 times and then the artificial respiration is performed twice (S110).

Meanwhile, when it is determined that the input signal is not input to the compression mode setting button, the control unit 320 may determine whether the input signal is input to the compression depth setting button (S111).

In this case, when it is determined that the input signal is input to the compression depth setting button (S111-YES), the control unit 320 may control the chest compression unit 310 so that the chest of the patient is compressed to at least one depth among 4 cm, 4.5 cm, 5 cm, and 5.5 cm (S112).

On the other hand, when it is determined that the input signal is not input to the compression depth setting button, the control unit 320 may determine whether the input signal is input to the compression speed setting button (S113).

In this case, when it is determined that the input signal is input to the compression speed setting button (S113-YES), the control unit 320 may control the chest compression unit 310 so that the chest of the patient is compressed at least one of 100, 110, or 120 times (S114).

On the other hand, when it is determined that the input signal is not input to the compression speed setting button (S113-NO), the control unit 320 may control to perform the treatment of the patient using the system combining a pulmonary resuscitation device and an automated external defibrillator of the present invention through the operation flow illustrated in FIG. 4.

A detailed description of preferred exemplary embodiments of the invention disclosed as described above is provided to enable a person skilled in the art to implement or practice the invention. Although preferred exemplary embodiments of the present invention have been disclosed above, it may be understood by those skilled in the art that the present invention may be variously modified and changed without departing from the scope of the present invention. For example, a person skilled in the art may use each configuration described in the above-described exemplary embodiments by combining them with each other. Accordingly, the present invention is not intended to be limited to the exemplary embodiments illustrated herein but is to be given the widest scope consistent with the principles and novel features disclosed herein.

The present invention may be implemented in another specific form without departing from claims. Therefore, the above-mentioned detailed description is to be interpreted as being illustrative rather than being restrictive in all aspects. The present invention is not intended to be limited to the exemplary embodiments illustrated herein but is to be given the widest scope consistent with the principles and novel features disclosed herein. In addition, claims that do not have an explicit reference relationship in the patent claims can be combined to form an exemplary embodiment.

### [Industrial Applicability]

The system combining a cardiopulmonary resuscitation device and an automated external defibrillator and an algorithm for controlling the same have excellent versatility as it is possible to provide chest compressions and electric shock to a patient with one system through a combination of a cardiopulmonary resuscitation device and an automated external defibrillator, and can accurately compress a chest of a patient through the sliding movement of the cardiopulmonary resuscitation device, and therefore, have industrial applicability.

## Claims

1. A system combining a cardiopulmonary resuscitation device and an automated external defibrillator, comprising:
a support plate (100) that supports a back of a patient;
a support (200) whose one end and the other end are coupled to both edges of the support plate (100); and
a hood (300) that is coupled to one side of the support (200), connected to a chest compression unit (310) for compressing a chest of the patient, and provided with a control unit (320) equipped with a shock button, **characterized in that**
the control unit (320) controls at least one of a chest compression unit (310), a electrocardiogram measurement unit (130), a rhythm determination and shock signal generation unit (140), and a electric shock unit (150) to operate,
the support plate (100) includes:
an electrocardiogram measurement unit (130) that detects an electrocardiogram signal from the patient, amplifies the detected electrocardiogram signal, removes noise from the amplified electrocardiogram signal, and then converts the electrocardiogram signal into a digital signal;
when receiving the digital signal from the electrocardiogram measurement unit (130), a rhythm determination and shock signal generation unit (140) that analyzes a heart rhythm of the patient with the digital signal to determine whether the heart rhythm of the patient is a shockable rhythm or a nonshockable rhythm, and generates an electric shock signal when the heart rhythm of the patient is the shockable rhythm; and
when receiving the electric shock signal from the rhythm determination and shock signal generation unit (140), an electric shock unit (150) that emits voltage energy to electrodes or pads attached to the chest of the patient to apply an electric shock to the patient;
the system combining a cardiopulmonary resuscitation device and an automated external defibrillator controls by performing
a first step in which the control unit (320) determines whether the system combining a cardiopulmonary resuscitation device and an automated external defibrillator is set to an automated external defibrillator mode and/or a chest compression mode;
a second step in which the control unit (320) determines whether a plurality of electrodes or pads of an electrocardiogram measurement unit (130) is attached to a chest of a patient;
a 2-1-th step in which, when the control unit (320) determines that the electrode or pad is attached to the chest of the patient, in order to compress the chest of the patient, a chest compression unit (310) is lowered to and stopped at an initial position where a lower end of the chest compression unit (310) and the chest of the patient are spaced apart by 1 cm;
a third step in which a rhythm determination and shock signal generation unit (140) analyzes a heart rhythm of the patient through the electrocardiogram measurement unit (130) to determine whether the heart rhythm is a shockable rhythm or a nonshockable rhythm;
a fourth step in which, after the heart rhythm of the patient is analyzed, the chest compression unit (310) is lowered to be in contact with the chest of the patient and presses the chest of the patient;
a fifth step in which the control unit (320) determines whether an input signal is input to a shock button when the rhythm determination and shock signal generation unit (140) determines that the heart rhythm of the patient is the shockable rhythm;
a sixth step in which, when the input signal is input to the shock button, the electric shock unit (150) generates high voltage energy through the rhythm determination and shock signal generation unit (140) to apply an electric shock to the patient; and
a seventh step in which, when the control unit (320) determines that no input signal is input to the shock button, the control unit (320) controls the electric shock unit (150) to internally discharge the high voltage energy,
wherein after the chest compression of the patient is performed in the fourth step, the second step, the 2-1-th step, the third step, the fourth step, the fifth step, the sixth step, and the seventh step are repeated.

2. The system of claim 1, wherein the first step includes:
a 1-1-th step in which a control button of the control unit (320) is input;
a 1-2-th step in which the control unit (320) determines whether the input signal is input to a rhythm analysis button for operating the electrocardiogram measurement unit (130) and the rhythm determination and shock signal generation unit (140); and
a 1-3-th step in which the control unit (320) determines whether the input signal is input to the shock button.

3. The system of claim 2, wherein the first step further includes a 1-4-th step in which the control unit (320) controls a chest compression treatment method for the patient when it is determined that the input signal is not input to the shock button.

4. The system of claim 3, wherein the 1-4-th step includes:
a step of setting a compression mode of the chest compression unit (310) to a compression continuous mode continuously compressing the chest of the patient or a compression 30 : 2 mode simultaneously performing the chest compression 30 times and artificial respiration twice on the patient through a compression mode setting button of the control unit;
a step of setting a compression depth of the chest compression unit (310) through a compression depth setting button of the control unit; and
a step of setting a compression speed of the chest compression unit (310) through a compression speed setting button of the control unit.

5. The system of claim 4, wherein in the fourth step, the chest compression unit (310) compresses the chest of the patient at the compression depth and compression speed set through the compression depth setting button and the compression speed setting button based on the compression continuous mode or the compression 30 : 2 mode set through the compression mode setting button.

6. The system of claim 1, wherein the third step includes:
a step of detecting, by the electrocardiogram measurement unit, an electrocardiogram signal from the patient through the plurality of electrodes or pads attached to the chest of the patient, amplifying the detected electrocardiogram signal, removing noise from the amplified electrocardiogram signal, and then converting the electrocardiogram signal into a digital signal; and
a step of analyzing, by the rhythm determination and shock signal generation unit (140), the heart rhythm of the patient based on the digital signal received from the electrocardiogram measurement unit (130).

7. The system of claim 1, wherein the sixth step includes:
a step of receiving, by the electric shock unit (150), from the rhythm determination and shock signal generation unit (140), an electric shock signal generated when the rhythm determination and shock signal generation unit (140) determines that the heart rhythm of the patient is the shockable rhythm; and
a step of generating, by the electric shock unit (150), high voltage energy based on the electric shock signal to apply the electric shock to the patient when the input signal is input to the shock button.

8. The system of claim 1, wherein in the seventh step, when the input signal is not input to the shock button, a voice guide is output from an alarm unit to inform that the input signal is not input to the shock button and the electric shock unit (150) internally discharges the high voltage energy.

9. The system of claim 8, wherein in the seventh step, when the input signal is not input to the shock button, from the alarm unit, the voice guide is output to induce the input of the input signal to the shock button, and then the voice guide indicating that the automated external defibrillator mode is canceled is output, the electric shock unit internally discharges the high voltage energy.

10. The system of claim 1, wherein the chest compression and electric shock are alternately or simultaneously applied to the patient depending on operation timing of the electric shock unit (150) and the chest compression unit (310).

11. The system of claim 1, wherein in a repetitive process of measuring the electrocardiogram of the patient, determining the rhythm, providing the chest compression and the electric shock, and internally discharging a high voltage energy, the control unit (320) controls the chest compression unit (310) to stop the chest compression immediately before the rhythm determination and shock signal generation unit (140) determines the rhythm and to continuously perform the chest compression immediately after the rhythm determination and shock signal generation unit (140) determines the rhythm.

12. The system of any one of the preceding claims, wherein the control unit performs the following steps:
step S1 (S1) in which settings of the system combining a cardiopulmonary resuscitation device and an automated external defibrillator are initialized and a self-test to determine whether the system combining a cardiopulmonary resuscitation device and an automated external defibrillator operates normally is performed;
step S5 (S5) in which, when the self-test is completed, an alarm unit built into the system combining a cardiopulmonary resuscitation device and an automated external defibrillator outputs a voice guide;
steps S7/S8 (S7, S8) in which when it is determined that the plurality of electrodes or pads is not attached to the chest of the patient, the alarm unit outputs the voice guide instructing to attach the plurality of electrodes or pads to the chest of the patient, and when it is determined that the plurality of electrodes or pads is attached to the chest of the patient, the chest compression unit (310) is lowered to an initial position for compressing the chest of the patient.
step S9 (S9) in which, after the chest compression unit (310) is lowered, the alarm unit outputs the voice guide to warn a user not to touch the patient during a treatment process of the patient;
step S14 (S14) in which, when it is determined that the heart rhythm of the patient is the shockable rhythm, the alarm unit outputs the voice guide instructing to input the shock button because an electric shock treatment is required for the patient;
step S17 (S17) in which, when it is determined that the input signal is not input to the shock button within a preset time, the alarm unit outputs the voice guide instructing to input the input signal to the shock button because the input signal is not input to the shock button; and
step S110 (S110) in which, when it is determined that the input signal is input to the compression mode setting button, it is determined whether a compression mode of the chest compression unit (310) is a compression continuous mode, when it is determined that the compression mode of the chest compression unit (310) is the compression continuous mode, the chest compression unit (310) is controlled to operate in the compression continuous mode, and when the compression mode of the chest compression unit (310) is not the compression continuous mode, the chest compression unit (310) is controlled in the compression mode that performs a specific number of times of artificial respirations after compressing the chest of the patient a specific number of times.

## Patentansprüche

1. Ein System, das ein Herz-Lungen-Wiederbelebungsgerät und einen automatisierten externen Defibrillator kombiniert, umfassend:
eine Stützplatte (100), die den Rücken eines Patienten stützt;
eine Stütze (200), deren eine Ende und das andere Ende an beiden Kanten der Stützplatte gekoppelt sind (100); und
eine Haube (300), die an eine Seite der Stütze (200) angeschlossen ist, die mit einer Herzdruckeinheit (310) zur Kompression der Brust des Patienten verbunden ist und mit einer Steuereinheit (320) ausgestattet ist, die mit einem Schockknopf ausgestattet ist, **gekennzeichnet dadurch, dass**
die Steuereinheit (320) mindestens eines von einer Brustkompressionseinheit (310), einer Elektrokardiogramm-Messeinheit (130), eine Rhythmusbestimmung- und Schocksignalerzeugungseinheit (140) sowie einer Elektroschockeinheit (150) zur Bedienung steuert,
die Stützplatte (100) umfasst:
eine Elektrokardiogramm-Messeinheit (130), die ein Elektrokardiogramm-Signal des Patienten detektiert, das detektierte Elektrokardiogramm-Signal verstärkt, Rauschen aus dem verstärkten Elektrokardiogramm-Signal entfernt und das Elektrokardiogramm-Signal dann in ein digitales Signal umwandelt;
eine Rhythmusbestimmung- und Schocksignalerzeugungseinheit (140), die bei Empfang des digitalen Signals von der Elektrokardiogramm-Messeinheit (130) den Herzrhythmus des Patienten anhand des digitalen Signals analysiert, um zu bestimmen, ob der Herzrhythmus des Patienten ein schockbarer Rhythmus oder ein nicht schockbarer Rhythmus ist, und ein elektrisches Schocksignal erzeugt, wenn der Herzrhythmus des Patienten ein schockbarer Rhythmus ist; und
eine Elektroschockeinheit (150), die bei Empfang des elektrischen Schocksignals von der Rhythmusbestimmungs- und Schocksignalerzeugungseinheit (140) die Spannungsenergie an Elektroden oder Polster abgibt, die am Brustkorb des Patienten befestigt sind, um einen elektrischen Schock auf den Patienten auszuüben;
das System, das ein Herz-Lungen-Wiederbelebungsgerät und einen automatisierten externen Defibrillator kombiniert, steuert die Durchführung
eines ersten Schritts, bei dem die Steuereinheit (320) feststellt, ob das System, das ein Herz-Lungen-Beatmungsgerät und einen automatisierten externen Defibrillator kombiniert, auf einen automatischen externen Defibrillator-Modus und/oder einen Kompressionsmodus für den Brustkorb eingestellt ist;
eines zweiten Schritts, bei dem die Kontrolleinheit (320) bestimmt, ob mehrere Elektroden oder Pads einer Elektrokardiogramm-Messeinheit (130) an der Brust eines Patienten befestigt sind;
eines 2-1. Schritts, bei dem eine Brustkompressionseinheit (310) in eine Ausgangsposition abgesenkt und gestoppt wird, wobei das untere Ende der Brustkompressionseinheit (310) und die Brust des Patienten um 1 cm voneinander entfernt sind, wenn die Steuereinheit (320) feststellt, dass die Elektrode oder das Pad am Brustkorb des Patienten befestigt ist, um die Brust des Patienten zu komprimieren;
eines dritten Schritts, bei dem eine Rhythmusbestimmungs- und Schocksignalgenerierungseinheit (140) den Herzrhythmus des Patienten anhand der Elektrokardiogramm-Messeinheit (130) analysiert, um festzustellen, ob der Herzrhythmus ein schockbarer oder nicht-schockbarer Rhythmus ist;
eines vierten Schritts, bei dem nach Analyse des Herzrhythmus des Patienten die Brustkompressionseinheit (310) so abgesenkt wird, dass sie mit der Brust des Patienten in Kontakt kommt und die Brust des Patienten drückt;
eines fünften Schritts, bei dem die Steuereinheit (320) bestimmt, ob ein Eingangssignal an einen Schockknopf eingegeben wird, wenn die Rhythmusbestimmungs- und Schocksignalgenerierungseinheit (140) bestimmt, dass der Herzrhythmus des Patienten der schockbare Rhythmus ist;
eines sechsten Schritts, bei dem, wenn das Eingangssignal an den Schockknopf eingegangen ist, die elektrische Schockeinheit (150) durch die Rhythmusbestimmungs- und Schocksignalerzeugungseinheit (140) Hochspannungsenergie erzeugt, um dem Patienten einen elektrischen Schock zuzufügen; und
eines siebten Schritts, bei dem die Steuereinheit (320), wenn sie feststellt, dass kein Eingangssignal an den Schockknopf eingegangen ist, die elektrische Schockeinheit (150) steuert, um die Hochspannungsenergie intern zu entladen,
wobei nach der Brustkompression des Patienten im vierten Schritt der zweite, der 2-1., der dritte, vierte, fünfte, sechste und siebte Schritt wiederholt werden.

2. Das System nach Anspruch 1, wobei der erste Schritt umfasst:
einen 1-1 Schritt, in dem eine Steuertaste der Steuereinheit (320) betätigt wird;
einen 1-2-Schritt, in dem die Steuereinheit (320) bestimmt, ob das Eingangssignal an einer Rhythmusanalyse-Taste zum Betreiben der Elektrokardiogramm-Messeinheit (130) und der Rhythmusbestimmung- und Schocksignalerzeugungseinheit (140) eingegeben wird; und
einen 1-3-Schritt, in dem die Steuereinheit (320) bestimmt, ob das Eingangssignal an die Schocktaste eingegeben wird.

3. Das System nach Anspruch 2, wobei der erste Schritt ferner einen 1-4-ten Schritt umfasst, in dem die Steuereinheit (320) eine Brustkompressionsbehandlung für den Patienten steuert, wenn festgestellt wird, dass das Eingangssignal nicht an den Schockknopf eingegeben wird.

4. Das System nach Anspruch 3, wobei der 1-4-te Schritt umfasst:
einen Schritt zum Einstellen eines Kompressionsmodus der Brustkompressionseinheit (310) auf einen Kompressions-Dauerbetrieb, der die Brust des Patienten kontinuierlich komprimiert, oder einen Kompressions-30:2-Modus, der gleichzeitig 30 Mal eine Brustkompression und zweimal eine künstliche Beatmung am Patienten durchführt, über eine Kompressionsmodus-Einstellungstaste der Steuereinheit;
einen Schritt zum Einstellen einer Kompressionstiefe der Brustkompressionseinheit (310) über eine Kompressionstiefeneinstellungstaste der Steuereinheit; und
einen Schritt zum Einstellen einer Kompressionsgeschwindigkeit der Brustkompressionseinheit (310) über eine Kompressionsgeschwindigkeitseinstellungstaste der Steuereinheit.

5. Das System nach Anspruch 4, wobei in dem vierten Schritt die Brustkompressionseinheit (310) die Brust des Patienten mit der Kompressionstiefe und Kompressionsgeschwindigkeit komprimiert, die über die Kompressionstiefeneinstelltaste und die Kompressionsgeschwindigkeitseinstelltaste auf der Grundlage des über die Kompressionsmoduseinstelltaste eingestellten kontinuierlichen Kompressionsmodus oder des 30:2-Kompressionsmodus eingestellt wurden.

6. Das System nach Anspruch 1, wobei der dritte Schritt umfasst:
einen Schritt, bei dem die Elektrokardiogramm-Messeinheit ein Elektrokardiogramm-Signal des Patienten über die mehreren Elektroden oder Pads, die an der Brust des Patienten angebracht sind, erfasst, das erfasste Elektrokardiogramm-Signal verstärkt, Rauschen aus dem verstärkten Elektrokardiogramm-Signal entfernt und dann das Elektrokardiogramm-Signal in ein digitales Signal umwandelt; und
einen Schritt des Analysierens des Herzrhythmus des Patienten durch die Rhythmusbestimmung- und Schocksignalerzeugungseinheit (140) auf der Grundlage des von der Elektrokardiogramm-Messeinheit (130) empfangenen digitalen Signals.

7. Das System nach Anspruch 1, wobei der sechste Schritt umfasst:
einen Schritt des Empfangens eines Elektroschocksignals durch die Elektroschockeinheit (150) von der Rhythmusbestimmungseinheit und Schocksignalerzeugungseinheit (140), das erzeugt wird, wenn die Rhythmusbestimmungs- und Schocksignalerzeugungseinheit (140) feststellt, dass der Herzrhythmus des Patienten ein schockbarer Rhythmus ist; und
einen Schritt des Erzeugens von Hochspannungsenergie durch die ElektroschockEinheit (150) auf der Grundlage des Elektroschocksignals, um den Elektroschock an den Patienten abzugeben, wenn das Eingangssignal an den Schockknopf eingegeben wird.

8. Das System nach Anspruch 1, wobei im siebten Schritt, wenn das Eingangssignal nicht an den Schockknopf eingegeben wird, eine Sprachführung von einer Alarmeinheit ausgegeben wird, um mitzuteilen, dass das Eingangssignal nicht an den Schockknopf eingegangen ist, und die Elektroschockeinheit (150) die Hochspannungsenergie intern entlädt.

9. Das System nach Anspruch 8, wobei im siebten Schritt, wenn das Eingangssignal nicht an den Schockknopf gegangen ist, von der Alarmeinheit die Sprachführung ausgegeben wird, um die Eingabe des Eingangssignals an den Schockknopf zu veranlassen, und dann die Sprachführung ausgegeben wird, die anzeigt, dass der automatisierte externe Defibrillatormodus abgebrochen wird, und die Elektroschockeinheit die Hochspannungsenergie intern entlädt.

10. Das System nach Anspruch 1, wobei die Brustkompression und der Elektroschock dem Patienten abwechselnd oder gleichzeitig verabreicht werden, abhängig vom Betriebszeitpunkt der Elektroschockeinheit (150) und der Brustkompressionseinheit (310).

11. Das System nach Anspruch 1, wobei in einem sich wiederholenden Prozess des Messens des Elektrokardiogramms des Patienten, des Bestimmens des Rhythmus, des Durchführens der Brustkompression und des Elektroschocks und des internen Entladens einer Hochspannungsenergie die Steuereinheit (320) die Brustkompressionseinheit (310) so steuert, dass die Brustkompression unmittelbar vor der Rhythmusbestimmung durch die Rhythmusbestimmungs- und Schocksignalgenerierungseinheit (140) gestoppt wird, und dass die Brustkompression unmittelbar nach der Rhythmusbestimmung durch die Rhythmusbestimmungs- und Schocksignalgenerierungseinheit (140) kontinuierlich durchgeführt wird.

12. Das System nach einem der vorstehenden Ansprüche, wobei die Steuereinheit die folgenden Schritte ausführt:
Schritt S1 (S1), in dem die Einstellungen des Systems, das ein Herz-Lungen-Wiederbelebungsgerät und einen automatisierten externen Defibrillator kombiniert, initialisiert werden und ein Selbsttest durchgeführt wird, um festzustellen, ob das System, das ein Herz-Lungen-Wiederbelebungsgerät und einen automatisierten externen Defibrillator kombiniert, normal funktioniert;
Schritt S5 (S5), in dem nach Abschluss des Selbsttests eine in das System, das ein Herz-Lungen-Wiederbelebungsgerät und einen automatisierten externen Defibrillator kombiniert, integrierte Alarmeinheit eine Sprachführung ausgibt;
Schritte S7/S8 (S7, S8), in denen, wenn festgestellt wird, dass die mehreren Elektroden oder Pads nicht an der Brust des Patienten angebracht sind, die Alarmeinheit die Sprachführung ausgibt, die anweist, die mehreren Elektroden oder Pads an der Brust des Patienten anzubringen, und wenn festgestellt wird, dass die mehreren Elektroden oder Pads an der Brust des Patienten angebracht sind, die Brustkompressionseinheit (310) auf eine Ausgangsposition zum Komprimieren der Brust des Patienten abgesenkt wird.
Schritt S9 (S9), in dem die Alarmeinheit nach dem Absenken der Brustkompressionseinheit (310) die Sprachführung ausgibt, um einen Benutzer zu warnen, den Patienten während eines Behandlungsprozesses des Patienten nicht zu berühren;
Schritt S14 (S14), in dem die Alarmeinheit, wenn festgestellt wird, dass der Herzrhythmus des Patienten ein schockbarer Rhythmus ist, die Sprachansage ausgibt, die anweist, den Schockknopf zu drücken, da für den Patienten eine Elektroschockbehandlung erforderlich ist;
Schritt S17 (S17), in dem die Alarmeinheit, wenn festgestellt wird, dass das Eingangssignal nicht innerhalb einer voreingestellten Zeit in die Schocktaste eingegeben wird, die Sprachführung ausgibt, die anweist, das Eingangssignal in die Schocktaste einzugeben, da das Eingangssignal nicht in die Schocktaste eingegeben wurde; und
Schritt S110 (S110), in dem, wenn festgestellt wird, dass das Eingangssignal an die Kompressionsmodus-Einstelltaste gegeben wird, festgestellt wird, ob ein Kompressionsmodus der Brustkompressionseinheit (310) ein Kompressions-Dauerbetrieb ist, und wenn festgestellt wird, dass der Kompressionsmodus der Brustkompressionseinheit (310) der Kompressions-Dauerbetrieb ist, wird die Brustkompressionseinheit (310) so gesteuert, dass sie im kontinuierlichen Kompressionsmodus arbeitet, und wenn der Kompressionsmodus der Brustkompressionseinheit (310) nicht der kontinuierliche Kompressionsmodus ist, wird die Brustkompressionseinheit (310) in dem Kompressionsmodus gesteuert, der eine bestimmte Anzahl von künstlichen Beatmungen durchführt, nachdem die Brust des Patienten eine bestimmte Anzahl von Malen komprimiert wurde.

## Revendications

1. Un système combinant un dispositif de réanimation cardio-pulmonaire et un défibrillateur externe automatisé, comprenant :
une plaque de support (100) qui soutient le dos d'un patient ;
un support (200) dont une extrémité et l'autre extrémité sont couplées aux deux bords de la plaque de support (100) ; et
un capot (300) qui est couplé à un côté du support (200), relié à une unité de compression thoracique (310) pour comprimer la poitrine du patient, et muni d'une unité de commande (320) équipée d'un bouton de choc, **caractérisé en ce que**
l'unité de commande (320) commande au moins l'une des unités suivantes : une unité de compression thoracique (310), une unité de mesure d'électrocardiogramme (130), une unité de détermination du rythme et de génération de signal de choc (140), et une unité de choc électrique (150) pour fonctionner,
la plaque de support (100) comprend :
une unité de mesure d'électrocardiogramme (130) qui détecte un signal d'électrocardiogramme provenant du patient, amplifie le signal d'électrocardiogramme détecté, supprime le bruit du signal d'électrocardiogramme amplifié, puis convertit le signal d'électrocardiogramme en un signal numérique ;
lorsqu'i l reçoit le signal numérique provenant de l'unité de mesure d'électrocardiogramme (130), une unité de détermination du rythme et de génération de signal de choc (140) qui analyse le rythme cardiaque du patient à l'aide du signal numérique afin de déterminer si le rythme cardiaque du patient est un rythme choquable ou un rythme non choquable, et génère un signal de choc électrique lorsque le rythme cardiaque du patient est un rythme choquable ; et
lorsqu'elle reçoit le signal de choc électrique provenant de l'unité de détermination du rythme et de génération du signal de choc (140), une unité de choc électrique (150) qui émet de l'énergie électrique vers des électrodes ou des coussinets fixés sur la poitrine du patient afin d'appliquer un choc électrique au patient ;
le système combinant un dispositif de réanimation cardio-pulmonaire et un défibrillateur externe automatisé effectue un contrôle en exécutant
une première étape dans laquelle l'unité de contrôle (320) détermine si le système combinant un dispositif de réanimation cardio-pulmonaire et un défibrillateur externe automatisé est réglé sur un mode défibrillateur externe automatisé et/ou un mode compression thoracique ;
une deuxième étape dans laquelle l'unité de commande (320) détermine si une pluralité d'électrodes ou de tampons d'une unité de mesure d'électrocardiogramme (130) est fixée à la poitrine d'un patient ;
une deuxième étape dans laquelle, lorsque l'unité de commande (320) détermine que l'électrode ou le tampon est fixé sur la poitrine du patient, afin de comprimer la poitrine du patient, une unité de compression thoracique (310) est abaissée et arrêtée à une position initiale où une extrémité inférieure de l'unité de compression thoracique (310) et la poitrine du patient sont espacées de 1 cm ;
une troisième étape dans laquelle une unité de détermination du rythme et de génération de signal de choc (140) analyse le rythme cardiaque du patient à l'aide de l'unité de mesure d'électrocardiogramme (130) afin de déterminer si le rythme cardiaque est un rythme choquable ou un rythme non choquable ;
une quatrième étape dans laquelle, après analyse du rythme cardiaque du patient, l'unité de compression thoracique (310) est abaissée pour entrer en contact avec la poitrine du patient et appuie sur la poitrine du patient ;
une cinquième étape dans laquelle l'unité de commande (320) détermine si un signal d'entrée est entré dans un bouton de choc lorsque l'unité de détermination du rythme et de génération du signal de choc (140) détermine que le rythme cardiaque du patient est un rythme choquable ;
une sixième étape dans laquelle, lorsque le signal d'entrée est entré dans le bouton de choc, l'unité de choc électrique (150) génère une énergie à haute tension par l'intermédiaire de l'unité de détermination du rythme et de génération du signal de choc (140) afin d'appliquer un choc électrique au patient ; et
une septième étape dans laquelle, lorsque l'unité de commande (320) détermine qu'aucun signal d'entrée n'est entré dans le bouton de choc, l'unité de commande (320) commande l'unité de choc électrique (150) pour décharger en interne l'énergie haute tension,
dans lequel, après que la compression thoracique du patient a été effectuée à la quatrième étape, la deuxième étape, la 2-1-ième étape, la troisième étape, la quatrième étape, la cinquième étape, la sixième étape et la septième étape sont répétées.

2. Le système selon la revendication 1, dans lequel la première étape comprend :
une étape 1-1 dans laquelle un bouton de commande de l'unité de commande (320) est actionné ;
une étape 1-2 dans laquelle l'unité de commande (320) détermine si le signal d'entrée est entré dans un bouton d'analyse de rythme pour actionner l'unité de mesure d'électrocardiogramme (130) et l'unité de détermination de rythme et de génération de signal de choc (140) ; et
une étape 1-3 dans laquelle l'unité de commande (320) détermine si le signal d'entrée est entré dans le bouton de choc.

3. Le système selon la revendication 2, dans lequel la première étape comprend en outre une 1-4ème étape dans laquelle l'unité de commande (320) commande un traitement par compression thoracique pour le patient lorsqu'il est déterminé que le signal d'entrée n'est pas entré dans le bouton de choc.

4. Le système selon la revendication 3, dans lequel la 1-4ème étape comprend :
une étape consistant à régler un mode de compression de l'unité de compression thoracique (310) sur un mode de compression continue comprimant en continu le thorax du patient ou un mode de compression 30 :2 effectuant simultanément 30 compressions thoraciques et deux respirations artificielles sur le patient à l'aide d'un bouton de réglage du mode de compression de l'unité de commande ;
une étape consistant à régler la profondeur de compression de l'unité de compression thoracique (310) à l'aide d'un bouton de réglage de la profondeur de compression de l'unité de commande ; et
une étape consistant à régler la vitesse de compression de l'unité de compression thoracique (310) à l'aide d'un bouton de réglage de la vitesse de compression de l'unité de commande.

5. Le système selon la revendication 4, dans lequel, dans la quatrième étape, l'unité de compression thoracique (310) comprime le thorax du patient à la profondeur et à la vitesse de compression définies à l'aide du bouton de réglage de la profondeur de compression et du bouton de réglage de la vitesse de compression, en fonction du mode de compression continue ou du mode de compression 30 :2 défini à l'aide du bouton de réglage du mode de compression.

6. Le système selon la revendication 1, dans lequel la troisième étape comprend :
une étape consistant à détecter, à l'aide de l'unité de mesure d'électrocardiogramme, un signal d'électrocardiogramme provenant du patient à l'aide de la pluralité d'électrodes ou de coussinets fixés sur la poitrine du patient, à amplifier le signal d'électrocardiogramme détecté, à supprimer le bruit du signal d'électrocardiogramme amplifié, puis à convertir le signal d'électrocardiogramme en un signal numérique ; et
une étape consistant à analyser, à l'aide de l'unité de détermination du rythme et de génération de signal de choc (140), le rythme cardiaque du patient sur la base du signal numérique reçu de l'unité de mesure d'électrocardiogramme (130).

7. Le système selon la revendication 1, dans lequel la sixième étape comprend :
une étape consistant à recevoir, par l'unité de choc électrique (150), à partir de l'unité de détermination du rythme et de génération du signal de choc (140), un signal de choc électrique généré lorsque l'unité de détermination du rythme et de génération du signal de choc (140) détermine que le rythme cardiaque du patient est un rythme pouvant être choqué ; et
une étape consistant à générer, par l'unité de choc électrique (150), une énergie haute tension sur la base du signal de choc électrique afin d'appliquer le choc électrique au patient lorsque le signal d'entrée est entré dans le bouton de choc.

8. Le système selon la revendication 1, dans lequel, dans la septième étape, lorsque le signal d'entrée n'est pas entré dans le bouton de choc, un guide vocal est émis par une unité d'alarme pour informer que le signal d'entrée n'est pas entré dans le bouton de choc et l'unité de choc électrique (150) décharge en interne l'énergie haute tension.

9. Le système selon la revendication 8, dans lequel, à la septième étape, lorsque le signal d'entrée n'est pas entré dans le bouton de choc, à partir de l'unité d'alarme, le guide vocal est émis pour inciter à entrer le signal d'entrée dans le bouton de choc, puis le guide vocal indiquant que le mode défibrillateur externe automatisé est annulé est émis, l'unité de choc électrique décharge en interne l'énergie haute tension.

10. Le système selon la revendication 1, dans lequel la compression thoracique et le choc électrique sont appliqués alternativement ou simultanément au patient en fonction du moment de fonctionnement de l'unité de choc électrique (150) et de l'unité de compression thoracique (310).

11. Le système selon la revendication 1, dans lequel, dans un processus répétitif consistant à mesurer l'électrocardiogramme du patient, à déterminer le rythme, à fournir la compression thoracique et le choc électrique, et à décharger en interne une énergie à haute tension, l'unité de commande (320) commande l'unité de compression thoracique (310) pour arrêter la compression thoracique immédiatement avant que l'unité de détermination du rythme et de génération du signal de choc (140) ne détermine le rythme, et pour effectuer en continu la compression thoracique immédiatement après que l'unité de détermination du rythme et de génération du signal de choc (140) ait déterminé le rythme.

12. Le système de l'une quelconque des revendications précédentes, dans lequel l'unité de commande effectue les étapes suivantes :
étape S1 (S1) dans laquelle les réglages du système combinant un dispositif de réanimation cardio-pulmonaire et un défibrillateur externe automatisé sont initialisés et un autotest est effectué pour déterminer si le système combinant un dispositif de réanimation cardio-pulmonaire et un défibrillateur externe automatisé fonctionne normalement ;
étape S5 (S5) dans laquelle, lorsque l'autotest est terminé, une unité d'alarme intégrée au système combinant un dispositif de réanimation cardio-pulmonaire et un défibrillateur externe automatisé émet un guide vocal ;
étapes S7/S8 (S7, S8) dans lesquels, lorsqu'il est déterminé que la pluralité d'électrodes ou de coussinets n'est pas fixée sur la poitrine du patient, l'unité d'alarme émet le guide vocal indiquant de fixer la pluralité d'électrodes ou de coussinets sur la poitrine du patient, et lorsqu'il est déterminé que la pluralité d'électrodes ou de coussinets est fixée sur la poitrine du patient, l'unité de compression thoracique (310) est abaissée jusqu'à une position initiale pour comprimer la poitrine du patient.
étape S9 (S9) dans laquelle, après que l'unité de compression thoracique (310) a été abaissée, l'unité d'alarme émet le guide vocal pour avertir un utilisateur de ne pas toucher le patient pendant un processus de traitement du patient ;
étape S14 (S14) dans laquelle, lorsqu'il est déterminé que le rythme cardiaque du patient est un rythme pouvant être traité par choc électrique, l'unité d'alarme émet un message vocal demandant d'appuyer sur le bouton de choc électrique, car un traitement par choc électrique est nécessaire pour le patient ;
étape S17 (S17) dans laquelle, lorsqu'il est déterminé que le signal d'entrée n'est pas entré sur le bouton de choc dans un délai prédéfini, l'unité d'alarme émet le message vocal demandant d'entrer le signal d'entrée sur le bouton de choc, car le signal d'entrée n'est pas entré sur le bouton de choc ; et
étape S110 (S110) dans laquelle, lorsqu'il est déterminé que le signal d'entrée est entré sur le bouton de réglage du mode de compression, il est déterminé si un mode de compression de l'unité de compression thoracique (310) est un mode de compression continue, lorsqu'il est déterminé que le mode de compression de l'unité de compression thoracique (310) est le mode de compression continue, l'unité de compression thoracique (310) est commandée pour fonctionner en mode de compression continue, et lorsqu'il est déterminé que le mode de compression de l'unité de compression thoracique (310) n'est pas le mode de compression continue, l'unité de compression thoracique (310) est commandée en mode de compression qui effectue un nombre spécifique de respirations artificielles après avoir comprimé la poitrine du patient un nombre spécifique de fois.
